# EUROPÄISCHE PATENTSCHRIFT

(11) **EP 3 417 844 B1**
(45) Veröffentlichungstag und Bekanntmachung des Hinweises auf die Patenterteilung: **28.07.2021**
(21) Anmeldenummer: 18180379.2
(22) Anmeldetag: 10.04.2015
(51) Int. Cl.: A61K 8/24, A61K 8/36, A61K 8/362, A61K 8/365, A61K 8/41, A61K 8/55, A61Q 17/04

(54) **SONNENSCHUTZMITTEL MIT REDUZIERTER NEIGUNG ZUR TEXTILVERFLECKUNG IV**
SUNSCREEN WITH A REDUCED TENDENCY TO STAIN TEXTILES IV
PRÉPARATION D'ÉCRAN SOLAIRE À TENDANCE RÉDUITE À LA FORMATION DE TACHES SUR LES TEXTILES IV

(30) Priorität: 28.04.2014 DE 102014207924
(43) Veröffentlichungstag der Anmeldung: 26.12.2018
(62) Teilanmeldung aus: 15716489.8
(73) Patentinhaber: Beiersdorf AG, 20253 Hamburg (DE)
(72) Erfinder: Weinert, Katrin, 22763 Hamburg (DE); Reske, Kathrin, 2164 Buxtehude (DE); Schade, Tatjana, 25866 Mildstedt/Rosendahl (DE); Bleckmann, Andreas, 22926 Ahrensburg (DE)

(56) Entgegenhaltungen:
- US-A1- 2009 099 075
- US-A1- 2013 104 319

## Beschreibung

Die vorliegende Erfindung betrifft Verfahren und Verwendungen von Iminodisuccinat in einer kosmetische Zubereitung enthaltend (2-[-4-(Diethylamino)-2-hydroxybenzoyl] Benzoesäurehexylester (INCI: Diethylamino Hydroxybenzoyl Hexyl Benzoate).

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Zwar kennt der Fachmann die WO2011/101250, doch konnte diese Schrift nicht den Weg zur vorliegenden Erfindung weisen.
WO 2012078961 beschreibt chelatisierende Polymere zur Photostabilisierung von Avobenzone und Verminderung der Ausbildung von farbigen Komplexen bei Waschen in hartem Wasser, doch konnte diese Schrift ebenfalls nicht den Weg zur vorliegenden Erfindung weisen.
Darüber hinaus kennt der Fachmann grundsätzlich kosmetische Zubereitungen mit (2-[-4-(Diethylamino)-2-hydroxybenzoyl] Benzoesäurehexylester (INCI: Diethylamino Hydroxybenzoyl Hexyl Benzoate) und dem Komplexbildner EDTA. Die Wirkung zur Fleckenreduktion von EDTA ist im alkalischen Medium, wie es im Waschwasser vorliegt, jedoch begrenzt.
Ferner kennt der Fachmann die US 2009/099075 A1 und US 2013/104319 A1, die ebenfalls nicht den Weg zur vorliegenden Erfindung weisen konnten.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche UV-A-Filter wie 2-[-4-(Diethylamino)-2-hydroxybenzoyl] Benzoesäurehexylester zu entwickeln, welche sich leichter aus den mit der Zubereitung kontaminierten Textilen herauswaschen lassen.

Überraschend gelöst wird die Aufgabe durch ein Verfahren nach Anspruch 1 sowie durch eine Verwendung nach Anspruch 2.

Es ist erfindungsgemäß vorteilhaft, wenn die erfindungsgemäße Zubereitung (2-[-4-(Diethylamino)-2-hydroxybenzoyl] Benzoesäurehexylester (INCI: Diethylamino Hydroxybenzoyl Hexyl Benzoate) in einer Menge von 0,01 bis 10 Gewichts-%, bezogen auf das Gesamtgewicht der Zubereitung, enthält.
Es ist erfindungsgemäß bevorzugt, wenn die erfindungsgemäße Zubereitung (2-[-4-(Diethylamino)-2-hydroxybenzoyl] Benzoesäurehexylester (INCI: Diethylamino Hydroxybenzoyl Hexyl Benzoate) in einer Menge von 2 bis 5 Gewichts-%, bezogen auf das Gesamtgewicht der Zubereitung, enthält.
Es ist vorteilhaft im Sinne der vorliegenden Erfindung, wenn die erfindungsgemäße Zubereitung einen oder mehrere Komplexbildner b) in einer Gesamtmenge von 0,1 bis 3 Gewichts-%, bezogen auf das Gesamtgewicht der Zubereitung, enthält.

Es ist bevorzugt im Sinne der vorliegenden Erfindung, wenn die erfindungsgemäße Zubereitung einen oder mehrere Komplexbildner gewählt aus der Gruppe der Phosphonsäuren, Phosphorsäuren und Carbonsäuren mit weniger als 2 Stickstoffatomen und/oder deren Alkalisalze und/ oder deren Amin-N-Oxide. in einer Gesamtmenge von 0,5 bis 1,5 Gewichts-%, bezogen auf das Gesamtgewicht der Zubereitung, enthält.

Dabei ist es erfindungsgemäß bevorzugt, wenn als Komplexbildner neben Iminodisuccinat eine oder mehrere der Verbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Natriumpolyphosphat
- Tetranatriumpyrophosphat
- Hydroxamsäure
- Polygalacturonsäure
- Bernsteinsäure
- Ameisensäure
- Äpfelsäure
   und/oder deren Alkalisalze eingesetzt werden.

Als erfindungsgemäß vorteilhafte Alkalisalze gelten dabei Natrium- und Kaliumsalze, wobei die Natriumsalze erfindungsgemäß bevorzugt werden.

Erfindungsgemäß vorteilhafte Ausführungsformen der vorliegenden Erfindung zeichnen sich dadurch aus, dass das Gewichtsverhältnis von (2-[-4-(Diethylamino)-2-hydroxybenzoyl] Benzoesäurehexylester (INCI: Diethylamino Hydroxybenzoyl Hexyl Benzoate) zur Gesamtmenge an Komplexbildnern gewählt aus der Gruppe der Phosphonsäuren, Phosphorsäuren und Carbonsäuren mit weniger als 2 Stickstoffatomen und/oder deren Alkalisalze und/oder deren Amin-N-Oxide von 5:1 bis 5:3 beträgt.

Es ist erfindungsgemäß vorteilhaft, wenn die erfindungsgemäße Zubereitung in Form einer Emulsion vorliegt. Erfindungsgemäß bevorzugt ist es in einem solchen Fall, wenn die Zubereitung in Form einer ÖI-in-Wasser-Emulsion (O/W-Emulsion) vorliegt.

In einem solchen Fall sind die erfindungsgemäß vorteilhaften Ausführungsformen der vorliegenden Erfindung dadurch gekennzeichnet, dass die Zubereitung einen oder mehrere Emulgatoren gewählt aus der Gruppe der Verbindungen Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Polyglyceryl-3-methylglucosedistearat, Stearinsäure, Kaliumcetylphosphat, enthalten.

Es ist erfindungsgemäß vorteilhaft, wenn die erfindungsgemäße Zubereitung einen oder mehrere UV-Filterenthält , die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornyliden-methyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxy-siloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4-(tert.-Butyl)-4'-methoxydibenzoylmethan; 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine); 4,4',4"-(1,3,5-Triazin-2,4,6-triyltriimino)-tris-benzoesäure-tris(2-ethyl-hexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist dabei erfindungsgemäß besonders bevorzugt, wenn die Zubereitung frei ist von 3-(4-Methylbenzyliden)-campher und 2-Hydroxy-4-methoxybenzophenon (Oxybenzon).

Erfindungsgemäß vorteilhafte Ausführungsformen können dadurch erhalten werden, dass die Zubereitung Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

Es ist erfindungsgemäß vorteilhaft, wenn die erfindungsgemäße Zubereitung Phenoxyethanol und/oder Methylparaben enthält.

Es ist dabei erfindungsgemäß bevorzugt, wenn die Zubereitung frei ist von Propyl- und Butylparaben, 3-lod-2-propinylbutylcarbamat,
Erfindungsgemäß vorteilhafte Ausführungsformen sind auch dadurch gekennzeichnet, dass die Zubereitung einen oder mehrere Wirkstoffe gewählt aus der Gruppe der Verbindungen Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

Die erfindungsgemäße Zubereitung kann ferner vorteilshaft Glycerin und/oder Ethanol enthalten. In einem solchen Falle ist eine Glycerin-Konzentration von 0,01 bis 10 Gewichts-%, bezogen auf das Gesamtgewicht der Zubereitung, erfindungsgemäß vorteilhaft. Für Ethanol liegt der erfindungsgemäß vorteilhafte Einsatzbereich zwischen 0,01 und 10 Gewichts-%, bezogen auf das Gesamtgewicht der Zubereitung.

Die Ölphase der erfindungsgemäßen Zubereitung kann darüber hinaus noch weitere Öl-, Fett- und Wachskomponenten enthalten, beispielsweise polaren Öle aus der Gruppe der Lecithine oder Verbindungen wie z. B. Cocoglycerid, Capryl/Caprinsäure Triglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Auch Verbindungen wie Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat können eingesetzt werden.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dialkylether und Dialkylcarbonate, vorteilhaft sind z. B. Dicaprylylether *(Cetiol OE)* und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn die Ölphase der erfindungsgemäßen Formulierungen einen Gehalt an C₁₂-₁₅-Alkylbenzoat aufweist.

Vorteilhafte Ölkomponenten sind ferner z. B. Isopropylpalmitat, Myristylmyristat, Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Erfindungsgemäß vorteilhafte Ausführungsformen der vorliegenden Erfindung sind auch dadurch gekennzeichnet, dass die Emulsion Dimethicone und/oder Cyclomethicon enthält.

Als polymeren Filmbildner zur Erhöhung der Wasserfestigkeit kann die erfindungsgemäße Zubereitung erfindungsgemäß vorteilhaft Vinylpyrrolidon/Hexadecen Copolymer enthalten. Darüber hinaus ist der Zusatz Tapiokastärke erfindungsgemäß vorteilhaft.

Die erfindungsgemäße Zubereitung kann insbesondere vorteilhaft als Tagespflegeprodukt oder Sonnenschutzmittel eingesetzt werden.

Erfindungsgemäß ist auch ein Verfahren zur Erleichterung der Auswaschbarkeit von (2-[-4-(Diethylamino)-2-hydroxybenzoyl] Benzoesäurehexylester (INCI: Diethylamino Hydroxybenzoyl Hexyl Benzoate) enthaltenden kosmetischen Zubereitungen aus Textilien, welches dadurch gekennzeichnet ist, dass dem Kosmetikum ein oder mehrere erfindungsgemäße Komplexbildner zugesetzt werden.

Auch ist das Verfahren zur Reduzierung der durch UV-Lichtschutzfilter ((2-[-4-(Diethylamino)-2-hydroxybenzoyl] Benzoesäurehexylester (INCI: Diethylamino Hydroxybenzoyl Hexyl Benzoate)) enthaltenden kosmetischen Zubereitungen hervorgerufenen Textilverfleckung, welches dadurch gekennzeichnet ist, dass dem Kosmetikum ein oder mehrere Komplexbildner zugesetzt werden, erfindungsgemäß.

Erfindungsgemäß ist ferner die Verwendung von erfindungsgemäßen Komplexbildnern in (2-[-4-(Diethylamino)-2-hydroxybenzoyl] Benzoesäurehexylester (INCI: Diethylamino Hydroxybenzoyl Hexyl Benzoate) enthaltenden kosmetischen Zubereitungen zur Erleichterung der Auswaschbarkeit der UV-Lichtschutzfilter aus mit den Zubereitungen kontaminierten Textilien, sowie die Verwendung von erfindungsgemäßen Komplexbildnern in UV-Lichtschutzfilter (insbesondere (2-[-4-(Diethylamino)-2-hydroxybenzoyl] Benzoesäurehexylester (INCI: Diethylamino Hydroxybenzoyl Hexyl Benzoate)) enthaltenden kosmetischen Zubereitungen zur Reduzierung der durch die Zubereitung hervorgerufene Textilverfleckung.

### Vergleichsversuch

Mit dem folgenden Versuch konnte der erfindungsgemäße Effekt beispielhaft belegt werden: Es wurde jeweils 1% der erfindungsgemäßen Hilfsstoffe zu einer Diethylamino Hydroxybenzoyl Hexylbenzoate enthaltenden Formulierung zugesetzt und die Verfleckungsreduzierende Wirkung (Reduktion b*) im Vergleich zu einer Formulierung ohne erfindungsgemäße Komplexbildner mittels beschriebener Methode bestimmt.

Als Beleg der verbesserten Auswaschbarkeit und verminderten Fleckenbildung der erfindungsgemäßen Zubereitungen wurden in vitro Untersuchungen durchgeführt, deren Ergebnisse in der Abbildung 1 und Tabelle 1 dargestellt sind.
Es wurden verschiedene Sonnenschutzemulsionen hinsichtlich der Bildung von gelben Flecke über einen in vitro Auftragungs-/ Waschzyklus untersucht. Es wurden dabei weiße vorgewaschene Baumwollmonitore (100% Baumwolle) verwendet. Dazu wurden je 25 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.
Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1 °C), 41% (±4%) rel. Luftfeuchte.
Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.
Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel, zehn Metallkugeln als Beiladung) und im Anschluss ein Spülvorgang (20°C, 15min, Leitu ngswasser).
Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.
Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle 1: ,getestete Zubereitungen und deren Gelbwert-Reduktion von Flecken; b*-Wert [%]**

| **INCI** | **Bsp.1** | **Bsp.2** |
|---|---|---|
| Tetranatriumiminodisuccinate | 1,0 | |
| Caprylic/Capric Triglyceride | 4,0 | 4,0 |
| Glyceryl Stearate | 1,0 | 1,0 |
| Hydrogenated Coco-Glycerides | 1,0 | 1,0 |
| Sodium Stearoyl Glutamate | 0,3 | 0,3 |
| Silica Dimethyl Silylate | 0,3 | 0,3 |
| Tapioka Stärke | 1,0 | 1,0 |
| Parfum | 0,5 | 0,5 |
| Glycerin | 0,9 | 0,9 |
| Phenoxyethanol | 0,6 | 0,6 |
| Methylparaben | 0,3 | 0,3 |
| Acrylates/C10-30 Alkyl Acrylate Crosspolymer | 0,15 | 0,15 |
| Xanthan Gummi | 0,4 | 0,4 |
| Alcohol Denat. | 8,0 | 8,0 |
| Trisnatrium EDTA | 0,2 | 0,2 |
| Octocrylene | 9,0 | 9,0 |
| Diethylamino Hydroxybenzoyl Hexyl Benzoate | 3,0 | 3,0 |
| Wasser ad. | 100 | ad. 100 |
| | | |
| Reduktion b*[%] | -15 | 0 |

Die Ergebnisse zeigen eine eindeutige Fleckenverminderung durch den Einsatz von Tetranatriumiminodisuccinate im Vergleich zur Formulierung ohne diesen erfindungsgemäßen Komplexbildner (Beispiel 2).

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **Beispiel [%]** | | | | |
|---|---|---|---|---|---|
| | **8** | **9** | **10** | **11** | **12** |
| Potassium Trisphosphonomethylamine Oxide | | 0,50 | | | 0,50 |
| Natriumpolyphosphat | | | 0,50 | | |
| Tetranatrium Pyrophosphat | | | | 0,50 | |
| Natriumdiethylenetriaminepentamethylenephosphonate | 0,50 | | | | 0,50 |
| Natrium EDTMP | | 0,50 | | | |
| Diethylentriaminpentamethylenphosphonsäure/ Salzsäure | | | 0,50 | | |
| Trisodium EDTA | 0,20 | 0,50 | 0,50 | 0,20 | 0,50 |
| Butyl methoxydibenzoylmethane | 5,00 | 3,00 | 3,00 | 5,00 | 4,50 |
| Diethylamino Hydroxybenzoyl Hexyl Benzoate | 3,00 | 3,50 | 5,00 | 4,50 | 3,50 |
| Phenylbenzimidazole Sulfonic Acid | | 1,00 | 1,00 | 1,00 | 1,00 |
| Ethylhexyl Salicylate | | 5,00 | 5,00 | 5,00 | |
| Titanium Dioxide | 3,00 | 2,00 | | | 3,00 |
| Trimethoxycaprylylsilane | 0,20 | 0,20 | | | 0,20 |
| Octocrylene | 10,00 | 10,00 | 10,00 | 10,00 | 9,00 |
| Homosalate | | 10,00 | 10,00 | 10,00 | 9,50 |
| Cetearyl Alcohol | | 1,00 | 0,50 | | |
| Xanthan Gum | 0,40 | 0,40 | 0,40 | | 0,40 |
| Acrylates/C10-30 Alkyl Acrylate Crosspolymer | 0,30 | 0,05 | 0,20 | 0,40 | 0,30 |
| Alcohol Denat. | 5,00 | 4,00 | 6,00 | 6,00 | 5,00 |
| Methylparaben | 0,30 | 0,30 | 0,30 | 0,30 | 0,30 |
| Phenoxyethanol | 0,60 | 0,60 | 0,60 | 0,60 | 0,60 |
| Citric Acid | 0,30 | | | | 0,30 |
| Sodium Citrate | 0,10 | | | | 0,10 |
| Glycerin | 3,00 | 9,00 | 3,00 | 9,00 | 3,00 |
| Parfum | | 0,40 | 0,60 | 0,30 | |
| VP/Hexadecene Copolymer | 0,50 | 0,50 | | 0,50 | 0,50 |
| Silica Dimethyl Silylate | | 0,50 | 0,50 | | |
| Sodium Cetearyl Sulfate | | 0,15 | | | |
| Glyceryl Stearate SE | | 1,00 | | | |
| Glyceryl Stearate Citrate | 2,00 | | | | 2,00 |
| Ceteareth-20 | | | | 1,00 | |
| Sodium Stearoyl Glutamate | | | 0,40 | | |
| Glyceryl Stearate | | | 1,00 | | |
| Hydrogenated Coco-Glycerides | 1,00 | | 1,00 | | 1,00 |
| C12-15 Alkyl Benzoate | 5,00 | | | | 5,00 |
| Myristyl Myristate | 1,00 | 1,00 | | | 1,00 |
| Stearyl Alcohol | 0,50 | | | | 0,50 |
| C18-36 Acid Triglyceride | | | | 0,50 | |
| C18-38 Alkyl Hydroxystearoyl Stearate | | 0,50 | | | |
| Isopropyl Stearate | 2,00 | | | | 2,00 |
| Butylene Glycol Dicaprylate/Dicaprate | 5,00 | | | 3,00 | 5,00 |
| Aqua | ad 100 | ad 100 | ad 100 | ad 100 | ad 100 |

## Patentansprüche

1. Verfahren zur Erleichterung der Auswaschbarkeit von (2-[-4-(Diethylamino)-2-hydroxybenzoyl] Benzoesäurehexylester (INCI: Diethylamino Hydroxybenzoyl Hexyl Benzoate), enthaltenden kosmetischen Zubereitungen aus Textilien, **dadurch gekennzeichnet, dass** dem Kosmetikum Iminodisuccinat zugesetzt wird.

2. Verwendung von Iminodisuccinat in (2-[-4-(Diethylamino)-2-hydroxybenzoyl] Benzoesäurehexylester (INCI: Diethylamino Hydroxybenzoyl Hexyl Benzoate), enthaltenden kosmetischen Zubereitungen zur Erleichterung der Auswaschbarkeit der UV-Lichtschutzfilter aus mit den Zubereitungen kontaminierten Textilien.

## Claims

1. Process for facilitating the ability of cosmetic preparations comprising hexyl 2-[4-(diethylamino)-2-hydroxybenzoyl]benzoate (INCI: Diethylamino Hydroxybenzoyl Hexyl Benzoate) to be washed out of textiles, **characterized in that** iminodisuccinate is added to the cosmetic.

2. Use of iminodisuccinate in cosmetic preparations comprising hexyl 2-[4-(diethylamino)-2-hydroxybenzoyl]benzoate (INCI: Diethylamino Hydroxybenzoyl Hexyl Benzoate) for facilitating the ability of the UV light protection filters to be washed out from textiles contaminated with the preparations.

## Revendications

1. Procédé pour la facilitation de la lessivabilité de préparations cosmétiques contenant de l'ester d'hexyle d'acide 2-[-4-(diéthylamino)-2-hydroxybenzoyl]benzoïque (INCI : Diethylamino Hydroxybenzoyl Hexyl Benzoate) sur des textiles, **caractérisé en ce que** de l'iminodisuccinate est ajouté au produit cosmétique.

2. Utilisation d'iminodisuccinate dans des préparations cosmétiques contenant de l'ester d'hexyle d'acide 2-[-4-(diéthylamino)-2-hydroxybenzoyl]benzoïque (INCI : Diethylamino Hydroxybenzoyl Hexyl Benzoate) pour la facilitation de la lessivabilité des filtres de protection contre les UV sur des textiles contaminés avec les préparations.
